(19) Europäisches Patentamt
European Patent Office
Office européen des brevets

(11) **EP 3 659 548 B1**

(12) **EUROPÄISCHE PATENTSCHRIFT**

(45) Veröffentlichungstag und Bekanntmachung des Hinweises auf die Patenterteilung:
**01.01.2025  Patentblatt 2025/01**

(21) Anmeldenummer: **18209308.8**

(22) Anmeldetag: **29.11.2018**

(51) Internationale Patentklassifikation (IPC):
**B32B 18/00** *(2006.01)*

(52) Gemeinsame Patentklassifikation (CPC):
**A61C 5/77; A61C 13/0006; A61C 13/083; C04B 35/486;** C04B 2235/3206; C04B 2235/3208; C04B 2235/3225; C04B 2235/3229; C04B 2235/6562; C04B 2235/6565; C04B 2235/6567; C04B 2235/6581; C04B 2235/6583; C04B 2235/6586; C04B 2235/661;                    (Forts.)

(54) **VERFAHREN ZUR HERSTELLUNG EINER DENTALEN RESTAURATION**

METHOD FOR PRODUCING A DENTAL RESTORATION

PROCÉDÉ DE FABRICATION D'UNE RESTAURATION DENTAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**03.06.2020  Patentblatt 2020/23**

(73) Patentinhaber: **Ivoclar Vivadent AG 9494 Schaan (LI)**

(72) Erfinder:
• **Rothbrust, Frank 6822 Röns (AT)**
• **Jiang, Bo 9470 Werdenberg (CH)**

• **Jussel, Rudolf 6800 Feldkirch (AT)**
• **Ritzberger, Christian 9472 Grabs (CH)**

(74) Vertreter: **Uexküll & Stolberg Partnerschaft von Patent- und Rechtsanwälten mbB Beselerstraße 4 22607 Hamburg (DE)**

(56) Entgegenhaltungen:
EP-A1- 0 853 938          EP-A1- 2 022 442
WO-A1-2015/200017     US-A1- 2008 303 181
US-A1- 2017 349 494

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
C04B 2235/77; C04B 2235/9653; C04B 2235/9661;
C04B 2235/9669; C04B 2237/348; C04B 2237/58

**Beschreibung**

Die vorliegende Erfindung betrifft ein Verfahren, welches ausgehend von einem Oxidkeramikmaterial die Herstellung einer dentalen Restauration mit hervorragenden Eigenschaften in kurzer Zeit ermöglicht. Die Erfindung betrifft auch die Verwendung eines Oxidkeramikmaterials zur Herstellung einer dentalen Restauration mittels des erfindungsgemäßen Verfahrens.

Zur Herstellung vollanatomischer Dentalrestaurationen werden häufig keramische Materialien wie Oxidkeramiken eingesetzt. Diese bieten eine hohe klinische Sicherheit, sind üblicherweise metallfrei, können auch bei minimal-invasiven Präparationen eingesetzt werden und sind preislich im Vergleich zu anderen metallfreien Restaurationen sehr attraktiv. Nachteilig sind jedoch die zahlreichen Arbeitsschritte, die meist zur Herstellung solcher Restaurationen erforderlich sind.

Üblicherweise werden die Restaurationen aus vorgesinterten Rohlingen herausgefräst oder geschliffen, gegebenenfalls eingefärbt, durch thermische Behandlung dichtgesintert und schließlich gegebenenfalls weiter eingefärbt, glasiert und/oder poliert.

Konventionelle Sinterverfahren für Dentalkeramiken beinhalten ein langsames Aufheizen auf eine Maximaltemperatur, bei der das eingesetzte Oxidkeramikmaterial dichtgesintert wird. Bedingt durch die geringe Aufheizgeschwindigkeit dauert ein solcher Sinterprozess typischerweise deutlich mehr als 4 Stunden und trägt dadurch erheblich zu einer vor allem bei Chairside-Behandlungen unbefriedigend großen Dauer des Produktionszyklus von Dentalkeramiken bei.

Ansätze zur Beschleunigung des Sinterprozesses durch Erhöhung der Aufheizgeschwindigkeit sind grundsätzlich bekannt.

So beschreibt EP 2 098 188 A1 einen Dentalofen und ein Verfahren zum Sintern von Dentalmaterialien, bei dem der Ofen in einer ersten Aufheizperiode mit einer schnellen Aufheizrate von mehr als 50 K/min auf eine Vorsintertemperatur von mindestens 1000°C aufgeheizt wird.

EP 2 101 133 A1 beschreibt einen Sinterofen und ein Verfahren zum Sintern von Dentalpräparaten, bei dem die Dentalpräparate entlang einer Sinterstrecke bewegt und dabei verschiedenen Temperaturen ausgesetzt werden. Dabei können in einem ersten Abschnitt hohe Aufheizraten von 300 K/min oder mehr eingesetzt werden.

WO 2012/057829 A2 beschreibt ein Verfahren zum schnellen Sintern von Keramik unter Verwendung von elektromagnetischer Induktion oder eines Plasmas.

WO 2015/091744 A1 beschreibt ein Verfahren zur Planung einer Sinterung eines Zahnersatzteils, bei dem in Abhängigkeit von bestimmten Geometrie- und Materialparametern des herzustellenden Zahnersatzteils ein Temperaturprofil für die Wärmebehandlung des Zahnersatzteils automatisch mittels eines Computers bestimmt wird. Dabei wird für die Sinterung bestimmter Zahnersatzteile eine Heizrate zwischen 100 K/min und 400 K/min verwendet.

WO 2015/121364 A1 beschreibt einen Sinterofen für Dentalbauteile mit einer Aufheizvorrichtung, die im Nutzbereich eine Aufheizrate von mindestens 200 K/min ermöglicht.

Auch die Sinterung von Dentalmaterialien unter Schutzgas oder im Vakuum ist bekannt.

WO 2011/020688 A1 beschreibt eine Vorrichtung zum sauerstofffreien Sintern von Metall oder Keramik in der Dentaltechnik unter Schutzgas.

EP 2 703 760 A1 beschreibt einen Dentalofen zum Sintern eines Zahnersatzes, dessen Heizraum entweder mittels einer konventionellen Verschlussvorrichtung wie etwa einer Tür oder mittels eines Aufsatzes mit einem Vakuumbehälter verschließbar ist und dadurch wahlweise zum normalen Sintern oder zum Vakuumsintern eingesetzt werden kann.

EP 2 022 442 A1 offenbart dentale Abutments und ein Verfahren zu deren Herstellung, bei dem aus einem binderhaltigen Oxidkeramikpulver Grünlinge geformt und diese durch zwei aufeinanderfolgende Wärmebehandlungen entbindert und gesintert werden. Diese Wärmebehandlungen können in Luft, im Vakuum oder in Gegenwart eines Gases wie Stickstoff oder Argon erfolgen. Dabei kann die Atmosphäre während des Sinterns in der Weise geändert werden, dass erst unter vermindertem Druck und später in einer inerten Atmosphäre gesintert wird.

WO 2015/200017 A1 beschreibt ein Verfahren zur Herstellung von Dentalrestaurationen, bei dem ein poröses Ausgangsmaterial gesintert wird, um eine gesinterte Lithiumdisilikat-Glaskeramik zu erhalten, wobei das Sintern oberhalb einer Temperatur von 600-700°C bei niedrigerem Druck als unterhalb dieser Temperatur erfolgt.

EP 0 853 938 A1 beschreibt Dentalrestaurationen aus Metall, die gewisse Mengen an Oxiden enthalten können. Zur Herstellung der Dentalrestaurationen wird ein Grünling in einer nicht-oxidierenden Atmosphäre entbindert und gesintert.

US 2017/349494 A1 beschreibt Zirkonoxid-Sinterkörper, die als dentale Restaurationsmaterialien verwendet werden können. Diese können unter anderem durch ein zweistufiges Sintern eines Grünkörpers hergestellt werden, bei dem das erste Sintern durch druckloses Sintern bei 1000°C bis weniger als 1650°C und das zweite Sintern bei mindestens 1650°C erfolgen kann. Um Sinterkörper mit hoher Dichte zu erhalten, kann beim zweiten Sintern heißisostatisches Pressen (HIP) mit Argon, Stickstoff oder Sauerstoff als Druckmedium eingesetzt werden, von denen Argon bevorzugt ist.

Es hat sich jedoch gezeigt, dass die bekannten Ansätze zur Beschleunigung des Sinterprozesses zu Keramikmaterialien führen, deren Eigenschaften insbesondere in optischer Hinsicht den hohen Anforderungen im Dentalbereich nicht genügen.

Der Erfindung liegt daher die Aufgabe zugrunde, ein Verfahren zur Herstellung einer dentalen Restauration bereitzustellen, mit dem in kurzer Zeit durch Sintern von Oxidkeramikmaterial dentale Restauration mit hervorragenden mechanischen und insbesondere optischen Eigenschaften hergestellt werden können.

Diese Aufgabe wird erfindungsgemäß gelöst durch das Verfahren zur Herstellung einer dentalen Restauration nach den Ansprüchen 1 bis 15. Gegenstand der Erfindung ist auch die Verwendung eines Oxidkeramikmaterials zur Herstellung einer dentalen Restauration nach Anspruch 16.

Das erfindungsgemäße Verfahren zur Herstellung einer dentalen Restauration zeichnet sich dadurch aus, dass ein Oxidkeramikmaterial

(a) einer ersten Wärmebehandlung unterworfen wird,
(b) einer zweiten Wärmebehandlung unterworfen wird und
(c) abgekühlt wird,

wobei die Wärmebehandlung in Schritt (a) bei niedrigerem Druck als die Wärmebehandlung in Schritt (b) erfolgt und die Wärmebehandlung in Schritt (b) in einer sauerstoffhaltigen Atmosphäre erfolgt

und wobei

die Wärmebehandlung in Schritt (a) bei einem Druck von weniger als 200 mbar erfolgt und/oder

in Schritt (b) das Oxidkeramikmaterial (b1) gegebenenfalls weiter aufgeheizt wird und (b2) bei einer Temperatur im Bereich von 1100 bis 1600°C gehalten und gesintert wird.

Es wurde überraschend gefunden, dass das erfindungsgemäße Verfahren eine sehr schnelle Sinterung von Oxidkeramiken zu dentalen Restaurationen ermöglicht, die gute mechanische Eigenschaften und insbesondere eine hohe Dichte aufweisen und zugleich auch in ästhetischer Hinsicht die hohen Anforderungen an dentale Restaurationen erfüllen und die optischen Eigenschaften von natürlichem Zahnmaterial ausgezeichnet imitieren können.

Bei dem in Schritt (a) eingesetzten Oxidkeramikmaterial handelt es sich üblicherweise um ein nicht dichtgesintertes und insbesondere um ein vorgesintertes Oxidkeramikmaterial. Üblicherweise weist das eingesetzte Oxidkeramikmaterial eine relative Dichte im Bereich von 30 bis 90%, insbesondere im Bereich von 40 bis 80% und bevorzugt im Bereich von 50 bis 70%, jeweils bezogen auf die Reindichte des Oxidkeramikmaterials, auf.

Die relative Dichte ist dabei das Verhältnis der scheinbaren Dichte des Oxidkeramikmaterials zur Reindichte des Oxidkeramikmaterials.

Die scheinbare Dichte des Oxidkeramikmaterials kann mittels der Immersionsmethode gemäß ISO 18754 aus der Masse des trockenen Probe ($m_t$), der scheinbaren Masse der in einer Immersionsflüssigkeit untergetauchten Probe ($m_i$) und der Dichte der Immersionsflüssigkeit ($\rho_i$) nach der Formel

$$\rho = \frac{m_t}{m_t - m_i} \times \rho_i$$

berechnet werden. Als Immersionsflüssigkeit wird vorzugsweise Tetrachlorkohlenstoff ($CCl_4$) eingesetzt.

Die Bestimmung der Reindichte des Oxidkeramikmaterials erfolgt, indem das Oxidkeramikmaterial auf ein Pulver mit einer mittleren Teilchengröße von 10 bis 30 $\mu$m, insbesondere von 20 $\mu$m, bezogen auf die Anzahl der Teilchen, gemahlen und die Dichte des Pulvers mittels Pyknometer ermittelt wird. Die Bestimmung der Teilchengröße kann beispielsweise mit dem CILAS® Particle Size Analyzer 1064 der Firma Quantachrome GmbH & Co. KG mittels Laserbeugung nach ISO 13320 (2009) durchgeführt werden.

In Schritt (a) wird das Oxidkeramikmaterial vorzugsweise auf eine Temperatur aufgeheizt, die im Bereich von 1100 bis 1600°C, insbesondere im Bereich von 1200 bis 1500°C, bevorzugt im Bereich von 1250 bis 1450°C und weiter bevorzugt im Bereich von 1300 bis 1400°C liegt und am meisten bevorzugt etwa 1350°C beträgt. Es ist weiter bevorzugt, dass das Oxidkeramikmaterial am Ende von Schritt (a) eine relative Dichte im Bereich von 90 bis 97%, insbesondere im Bereich von 93 bis 96% und bevorzugt eine relative Dichte von etwa 95%, jeweils bezogen auf die Reindichte des Oxidkeramikmaterials, aufweist.

Vorzugsweise wird das Oxidkeramikmaterial in Schritt (a) mit einer Heizrate im Bereich von 10 bis 500 K/min, insbesondere 50 bis 250 K/min und bevorzugt 100 bis 200 K/min aufgeheizt. In einer bevorzugten Ausführungsform wird

EP 3 659 548 B1

das Oxidkeramikmaterial zunächst mit einer Heizrate von 50 bis 500 K/min, insbesondere 100 bis 250 K/min und bevorzugt 150 bis 200 K/min auf eine Temperatur aufgeheizt, die 100 bis 700 K, insbesondere 200 bis 450 K und vorzugsweise etwa 350 K unterhalb der maximalen in Schritt (a) erreichten Temperatur liegt, und anschließend mit einer Heizrate von 10 bis 200 K/min, insbesondere 25 bis 150 K/min und bevorzugt 50 bis 100 K/min weiter aufgeheizt.

Die Wärmebehandlung in Schritt (a) erfolgt erfindungsgemäß bei niedrigerem Druck als die Wärmebehandlung in Schritt (b). Bevorzugt erfolgt die Wärmebehandlung in Schritt (a) bei einem Druck von weniger als 200 mbar, vorzugsweise weniger als 100 mbar und besonders bevorzugt weniger als 50 mbar, und insbesondere bei einem Druck im Bereich von 0,1 bis 200 mbar, vorzugsweise im Bereich von 1 bis 150 mbar und am besonders bevorzugt im Bereich von 50 bis 100 mbar.

Die Einstellung dieses Drucks kann bei Umgebungstemperatur erfolgen, bevor mit dem Aufheizen des Oxidkeramikmaterials begonnen wird. Alternativ kann das Oxidkeramikmaterial zunächst auf eine oberhalb der Umgebungstemperatur liegende Temperatur aufgeheizt werden, bevor der für Schritt (a) definierte Druck eingestellt wird. Diese Temperatur liegt vorzugsweise im Bereich von 20 bis 500°C und insbesondere im Bereich von 25 bis 100°C.

In Schritt (b) wird das Oxidkeramikmaterial vorzugsweise (b1) gegebenenfalls weiter aufgeheizt und (b2) bei einer, vorzugsweise konstanten, Temperatur im Bereich von 1100 bis 1600°C, insbesondere im Bereich von 1300 bis 1600°C und bevorzugt im Bereich von 1350 bis 1550°C und besonders bevorzugt bei einer Temperatur etwa 1500°C gehalten und gesintert. Dabei erfolgt das weitere Aufheizen in Schritt (b1) vorzugsweise mit einer Heizrate von 10 bis 200 K/min, insbesondere 25 bis 150 K/min und bevorzugt 50 bis 100 K/min. Das Halten in Schritt (b2) erfolgt vorzugsweise für 1 bis 60 Minuten, insbesondere 2 bis 30 Minuten, bevorzugt 3 bis 15 Minuten und besonders bevorzugt etwa 5 Minuten. Durch das Halten bei der entsprechenden Temperatur wird das Oxidkeramikmaterial typischerweise dichtgesintert. Danach weist das Oxidkeramikmaterial vorzugsweise eine relative Dichte von mindestens 97%, insbesondere mindestens 98%, bevorzugt mindestens 99% und am meisten bevorzugt mindestens 99,5%, jeweils bezogen auf die Reindichte des Oxidkeramikmaterials, auf.

Die Wärmebehandlung in Schritt (b) erfolgt erfindungsgemäß bei höherem Druck als die Wärmebehandlung in Schritt (a). Vorzugsweise wird die Wärmebehandlung in Schritt (b) bei einem Druck von mehr als 500 mbar und insbesondere bei Umgebungsdruck ausgeführt.

Die Wärmebehandlung in Schritt (b) erfolgt in einer sauerstoffhaltigen Atmosphäre. Als sauerstoffhaltige Atmosphäre kommen insbesondere Luft, mit Sauerstoff angereicherte Luft sowie Sauerstoff in Frage. Zur Einstellung einer solchen Atmosphäre kann die für die Wärmebehandlung verwendete Heizkammer mit Luft und/oder Sauerstoff gefüllt werden. In einer bevorzugten Ausführungsform wird die für die Wärmebehandlung verwendete Heizkammer während Schritt (b) diskontinuierlich oder vorzugsweise kontinuierlich mit einer sauerstoffhaltigen Atmosphäre, vorzugsweise Luft, mit Sauerstoff angereicherter Luft oder Sauerstoff, durchströmt, insbesondere mit einer Strömungsgeschwindigkeit von 0,1 bis 50 l/min, bevorzugt 1 bis 20 l/min und besonders bevorzugt 4 bis 8 l/min.

Außerdem ist es bevorzugt, dass das Oxidkeramikmaterial in Schritt (a) auf eine Temperatur aufgeheizt wird, die 0 bis 500 K, insbesondere 10 bis 250 K, bevorzugt 50 bis 200 K und besonders bevorzugt 100 bis 150 K unterhalb der Temperatur oder des Temperaturbereichs liegt, bei der/dem das Oxidkeramikmaterial in Schritt (b) gehalten wird.

Anschließend wird in Schritt (c) das Oxidkeramikmaterial abgekühlt. Vorzugsweise wird das Oxidkeramikmaterial auf eine Temperatur abgekühlt, die im Bereich von 20 bis 1300°C, insbesondere im Bereich von 100 bis 1250°C und bevorzugt im Bereich von 1000 bis 1200°C liegt. Nach Erreichen einer solchen Temperatur kann das Oxidkeramikmaterial aus der Heizkammer entfernt werden. Vorzugsweise erfolgt das Abkühlen mit einer Abkühlrate von 50 bis 200 K/min, insbesondere 75 bis 175 K/min und bevorzugt 100 bis 150 K/min.

Das nach dem erfindungsgemäßen Verfahren erhaltene Oxidkeramikmaterial weist vorzugsweise eine zahlenmittlere Korngröße im Bereich von 1 nm bis 1000 nm, insbesondere von 10 nm bis 800 nm und bevorzugt von 100 nm bis 600 nm auf. Die zahlenmittlere Korngröße kann insbesondere nach dem Linienschnittverfahren gemäß DIN EN 623-3 oder ASTM E 112 bestimmt werden, wobei der ermittelte Wert zur Umrechnung auf die reale zahlenmittlere Korngröße im dreidimensionalen Mikrogefüge nach M. I. Mendelson, J. Am. Ceram. Soc. 1969, 52(8), 443-446 mit einer Proportionalitätskonstante von 1,56 multipliziert wird.

Das erfindungsgemäße Verfahren eignet sich für verschiedene Arten von Oxidkeramikmaterialien. Oxidkeramikmaterialien sind allgemein hochkristalline Keramikmaterialien, die auf Oxidverbindungen basieren und allenfalls einen sehr geringen Anteil an Glasphase aufweisen. Typische Oxidkeramikmaterialien basieren auf $ZrO_2$, $Al_2O_3$, $TiO_2$, $MgO$, Kombinationen, Mischkristallen oder Kompositen davon, insbesondere $ZrO_2$/$Al_2O_3$ (ZTA), $Al_2O_3$/$ZrO_2$ (ATZ) oder $ZrO_2$/Spinell, wobei Spinell vorzugsweise Sr-Spinell, Mg-Spinell, La-Spinell und/oder Ce-Spinell ist. Oxidkeramikmaterialien auf Basis von $ZrO_2$ und/oder $Al_2O_3$ sind erfindungsgemäß bevorzugt.

Besonders bevorzugt sind Oxidkeramikmaterialien auf Basis von Zirkonoxid und insbesondere auf Basis von polykristallinem tetragonalem Zirkonoxid (TZP). Ganz besonders bevorzugt sind Oxidkeramikmaterialien auf Basis von Zirkonoxid, bei denen das Zirkonoxid mit $Y_2O_3$, $La_2O_3$, $CeO_2$, $MgO$ und/oder $CaO$ stabilisiert ist und vorzugsweise mit 2 bis 12 Mol-%, insbesondere 3 bis 5 Mol-%, dieser Oxide, bezogen auf den Gehalt an Zirkonoxid, stabilisiert ist.

Es ist weiter bevorzugt, dass das Oxidkeramikmaterial eingefärbt ist. Hierunter wird erfindungsgemäß ein Oxidkeramikmaterial verstanden, das mit einem oder mehreren färbenden Elementen versetzt ist. Beispiele für geeignete färbende Elemente sind Fe, Mn, Cr, Pr, Tb, Er, Yb, Ce, Co, Ni, Nd, Cu und Bi. Besonders bevorzugt umfasst das Oxidkeramikmaterial mindestens zwei Schichten, die sich in ihrer Farbe unterscheiden.

Im Sinne der vorliegenden Anmeldung beziehen sich die Begriffe "Farbe" und "eingefärbt" auf die Farbe, Helligkeit und/oder Transluzenz eines Materials.

"Transluzenz" ist die Lichtdurchlässigkeit eines Materials, Körpers oder einer Schicht, d.h. das Verhältnis von durchgelassener zu eingestrahlter Lichtintensität.

Farben können auch durch die Farbkoordinaten $L^*$, $a^*$ und $b^*$ im $L^*a^*b^*$-Farbraum oder durch einen in der Dentalindustrie üblicherweise verwendeten Farbcode charakterisiert werden. Im $L^*a^*b^*$-Farbraum beschreibt der Wert $L^*$ die Helligkeit einer Farbe mit Werten von 0 (schwarz) bis 100 (weiß), der Wert $a^*$ den Grün- oder Rotanteil einer Farbe, wobei negative Werte für Grün und positive Werte für Rot stehen, und der Wert $b^*$ den Blau- oder Gelbanteil einer Farbe, wobei negative Werte für Blau und positive Werte für Gelb stehen. Beispiele für in der Dentalindustrie übliche Farbcodes sind Vitapan classical® und Vita 3D Master®, beide von der VITA Zahnfabrik H. Rauter GmbH & Co. KG, und Chromascop® von der Ivoclar Vivadent AG. Die Transluzenz kann durch den Kontrastwert CR charakterisiert werden, wobei 0% vollständig transparent und 100% vollständig opak bedeutet.

Üblicherweise erfolgen die Bestimmung der Farbkoordinaten $L^*$, $a^*$ und $b^*$ nach DIN 5033 und DIN 6174 und die Bestimmung der Transluzenz nach BS 5612. Die entsprechenden Messungen können insbesondere mittels eines Spektrophotometers vom Typ CM-3700d (Konica-Minolta) durchgeführt werden. Hierzu werden für die Messungen Probenkörper eingesetzt, die mit Diamantpartikeln (Partikelgröße 15-20 pm) beidseitig nass beschliffen wurden, um eine finale Probendicke von 2,00 $\pm$ 0,025 mm zu erhalten.

Vorzugsweise liegen die Farbe oder die Farben der erfindungsgemäß erhaltenen dentalen Restauration im Bereich der Farben natürlicher Zähne. Besonders bevorzugt weisen die erfindungsgemäß erhaltenen dentalen Restauration einen $L^*$-Wert im Bereich von 50 bis 100, insbesondere im Bereich von 80 bis 97, einen $a^*$-Wert im Bereich von -10 bis 10, insbesondere im Bereich von -1 bis 5, einen $b^*$-Wert im Bereich von 0 bis 50, insbesondere im Bereich von 1 bis 20, und/oder einen CR-Wert im Bereich von 50 bis 100%, insbesondere im Bereich von 75 bis 99%, auf.

Das erfindungsgemäße Verfahren eignet sich in besonderer Weise für die Herstellung von dentalen Restaurationen. Besonders bevorzugte dentale Restaurationen sind Brücken, Inlays, Onlays, Kronen, Veneers, Schalen und Abutments. Ganz besonders eignet sich das erfindungsgemäße Verfahren zur Herstellung von dentalen Restaurationen, insbesondere Brücken, die zwei oder mehr Glieder umfassen.

Die Erfindung betrifft auch die Verwendung eines Oxidkeramikmaterials zur Herstellung einer dentalen Restauration, bei der das Oxidkeramikmaterial

(a) einer ersten Wärmebehandlung unterworfen wird,
(b) einer zweiten Wärmebehandlung unterworfen wird und
(c) abgekühlt wird,

wobei die Wärmebehandlung in Schritt (a) bei niedrigerem Druck als die Wärmebehandlung in Schritt (b) erfolgt und die Wärmebehandlung in Schritt (b) in einer sauerstoffhaltigen Atmosphäre erfolgt

und wobei

die Wärmebehandlung in Schritt (a) bei einem Druck von weniger als 200 mbar erfolgt und/oder

in Schritt (b) das Oxidkeramikmaterial (b1) gegebenenfalls weiter aufgeheizt wird und (b2) bei einer Temperatur im Bereich von 1100 bis 1600°C gehalten und gesintert wird.

Bevorzugte Ausführungsformen der Verwendung sind wie oben für das erfindungsgemäße Verfahren beschrieben.

Die Erfindung wird im Folgenden anhand von Beispielen näher erläutert.

Beispiele

Allgemeine Verfahrensweise: Herstellung von Prüfkörpern

Aus kommerziell erhältlichen Blöcken oder Scheiben aus Oxidkeramik wurden durch Trockensägen mit einer Diamantsäge Prüfkörper mit einer Höhe von 17 mm, einer Breite von 15,5 mm und einer Dicke von 2,5 bis 3,5 mm hergestellt. Nach dem Sägen wurden die Prüfkörper bei 80°C für 2 h in einem Trockenschrank getrocknet.

**Beispiel 1A**

Ein gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT BL, Ivoclar Vivadent) erhaltener Prüfkörper wurde in einem Sinterofen mit SiC-Heizelement gesintert. Dazu wurde der Prüfkörper bei Raumtemperatur in die Heizkammer des Sinterofens eingebracht, die Heizkammer verschlossen und in der Heizkammer ein partielles Vakuum mit einem finalen Druck von etwa 50 bis 100 mbar erzeugt. Der Prüfkörper wurde mit einer Heizrate von etwa 180 K/min auf eine Temperatur von etwa 1000°C und weiter mit einer Heizrate von etwa 80 K/min auf eine Temperatur von etwa 1350°C aufgeheizt. Bei Erreichen dieser Temperatur wurde die Heizkammer mit Frischluft geflutet und anschließend kontinuierlich mit einer Strömungsgeschwindigkeit von etwa 5 l/min mit Frischluft durchströmt, während der Prüfkörper mit einer Heizrate von etwa 80 K/min weiter auf eine Temperatur von 1500°C aufgeheizt, bei dieser Temperatur etwa 5 Minuten lang gehalten und dann mit einer Abkühlrate von etwa 140 K/min auf eine Temperatur von etwa 1100°C abgekühlt wurde. Anschließend wurde die Heizkammer geöffnet. Die Gesamtdauer des Sinterprozesses betrug etwa 19,5 min.

**Beispiel 1B (Vergleich)**

Beispiel 1A wurde wiederholt, wobei jedoch kein Vakuum erzeugt wurde, sondern die Heizkammer über die gesamte Dauer des Sinterprozesses kontinuierlich mit einer Strömungsgeschwindigkeit von etwa 5 l/min mit Frischluft durchströmt wurde.

**Beispiel 1C (Vergleich)**

Ein gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT BL, Ivoclar Vivadent) erhaltener Prüfkörper wurde in einem Sinterofen vom Typ Programat CS4 (Ivoclar Vivadent) mit dem Programm P2 ohne automatische Vortrocknung gesintert. Dabei wurde der Prüfkörper mit einer Heizrate von etwa 130 K/min von Raumtemperatur auf etwa 900°C, weiter mit einer Heizrate von etwa 50 K/min auf etwa 1035°C und schließlich mit einer Heizrate von etwa 15 K/min auf etwa 1460°C aufgeheizt. Bei dieser Temperatur wurde der Prüfkörper etwa 6 min lang gehalten und dann mit einer Abkühlrate von etwa 70 K/min auf eine Temperatur von etwa 1200°C abgekühlt. Anschließend wurde die Heizkammer geöffnet. Die Gesamtdauer des Sinterprozesses betrug etwa 47 min.

Die Dichte, Transluzenz und Farbkoordinaten der in den Beispielen 1A-C erhaltenen Oxidkeramikmaterialien sind in Tabelle 1 wiedergegeben.

Tabelle 1

| Beispiel | Sinterdauer [min] | Dichte [g/cm$^3$] | CR [%] | L* | a* | b* |
|---|---|---|---|---|---|---|
| 1A | 19, 5 | 6, 065 | 88, 99 | 95, 87 | -0,37 | 1,75 |
| 1B (Vergleich) | 19, 5 | 6, 066 | 92,54 | 96, 14 | -0,31 | 1, 81 |
| 1C (Vergleich) | 47 | 6, 068 | 88,53 | 95,79 | -0,27 | 1, 96 |

Danach ermöglicht es das erfindungsgemäße Verfahren gemäß Beispiel 1A, bei sehr kurzer Sinterdauer von unter 20 min eine mit den Beispielen 1B und 1C vergleichbare Dichte zu gelangen und dabei zugleich eine Transluzenz zu erhalten, die mit der bei langsamer Sinterung gemäß Beispiel 1C erhaltenen Transluzenz vergleichbar und deutlich höher als die bei schneller Sinterung gemäß Beispiel 1B erhaltene Transluzenz ist.

Beispiel 2

Gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT BL B45 oder IPS e.max ZirCAD LT A3 B45, Ivoclar Vivadent) bzw. aus kommerziell erhältlichen Oxidkeramik-Scheiben auf Basis von Zirkonoxid (Zenostar MT 3, Wieland Dental + Technik) erhaltene Prüfkörper wurden jeweils in einem Sinterofen mit $MoSi_2$-Heizelement gesintert. Dazu wurden die Prüfkörper bei Raumtemperatur in die Heizkammer des Sinterofens eingebracht, die Heizkammer verschlossen und in der Heizkammer ein partielles Vakuum mit einem finalen Druck von <50 mbar erzeugt. Die Prüfkörper wurden mit einer Heizrate von etwa 180 K/min auf eine Temperatur von etwa 1050°C und weiter mit einer Heizrate von etwa 80 K/min auf eine Temperatur von etwa 1500°C aufgeheizt. Gemäß der Tabelle 2 wurde die Heizkammer bei Erreichen einer Temperatur von 1350, 1400, 1450 bzw. 1500°C mit Frischluft geflutet und anschließend während des restlichen Sinterprozesses kontinuierlich mit einer Strömungsgeschwindigkeit von etwa 5 l/min mit Frischluft durchströmt. Bei einer Temperatur von etwa 1500°C wurden die Prüfkörper etwa 5 Minuten lang gehalten und dann mit einer Abkühlrate von etwa 140 K/min auf eine Temperatur von etwa 1100°C abgekühlt. Anschließend wurde die Heizkammer geöffnet.

Zum Vergleich wurde die obige Verfahrensweise wiederholt, wobei jedoch kein Vakuum erzeugt wurde, sondern die Heizkammer über die gesamte Dauer des Sinterprozesses kontinuierlich mit einer Strömungsgeschwindigkeit von etwa 5 l/min mit Frischluft durchströmt wurde.

Die Dichte, Transluzenz und Farbkoordinaten der jeweils erhaltenen Oxidkeramikmaterialien sind in Tabelle 2 wiedergegeben.

Tabelle 2

| Material | Vakuum bis [°C] | Dichte [g/cm$^3$] | CR [%] | L* | a* | b* |
|---|---|---|---|---|---|---|
| IPS e.max ZirCAD LT BL B45 | 1350 | 6,072 | 91,06 | 95,48 | -0,39 | 2,70 |
| | 1400 | 6,075 | 89,98 | 95,44 | -0,41 | 2,95 |
| | 1450 | 6,077 | 89,04 | 95,38 | -0,48 | 3,01 |
| | 1500 | 6,075 | 88,05 | 94,95 | -0,47 | 3,25 |
| | kein Vakuum* | 6,070 | 92,35 | 96,06 | -0,25 | 1,85 |
| IPS e.max ZirCAD LT A3 B45 | 1350 | 6,079 | 96,66 | 82,78 | 4,76 | 17,89 |
| | 1400 | 6,080 | 96,96 | 83,12 | 4,55 | 17,45 |
| | 1450 | 6,081 | 94,14 | 81,31 | 5,41 | 18,34 |
| | 1500 | 6,078 | 97,62 | 83,59 | 3,80 | 16,37 |
| | kein Vakuum* | 6,070 | 97,29 | 84,82 | 3,77 | 16,34 |
| Zenostar MT 3 | 1350 | 6,041 | 98,83 | 86,13 | 1,53 | 14,73 |
| | 1400 | 6,041 | 98,34 | 87,21 | 1,14 | 14,11 |
| | 1450 | 6,048 | 96,93 | 83,93 | 2,23 | 15,91 |
| | 1500 | 6,044 | 98,71 | 86,16 | 1,48 | 15,65 |
| | kein Vakuum* | 6,033 | 99,50 | 86,96 | 1,27 | 14,45 |
| * (Vergleich) | | | | | | |

**Beispiel 3**

Gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT A3 B45, Ivoclar Vivadent) bzw. aus kommerziell erhältlichen Oxidkeramik-Scheiben auf Basis von Zirkonoxid (Zenostar MT 3, Wieland Dental + Technik) erhaltene Prüfkörper wurden jeweils in einem Sinterofen mit $MoSi_2$-Heizelement gesintert. Dazu wurden die Prüfkörper bei Raumtemperatur in die Heizkammer des Sinterofens eingebracht, die Heizkammer verschlossen und gemäß Tabelle 3 in der Heizkammer ein partielles Vakuum mit einem finalen Druck von 50, 100 bzw. 200 mbar erzeugt. Die Prüfkörper wurden mit einer Heizrate von etwa 180 K/min auf eine Temperatur von etwa 1000°C und weiter mit einer Heizrate von etwa 80 K/min auf eine Temperatur von etwa 1350°C aufgeheizt. Bei Erreichen dieser Temperatur wurde die Heizkammer mit Frischluft geflutet und anschließend kontinuierlich mit einer Strömungsgeschwindigkeit von etwa 5 l/min mit Frischluft durchströmt, während die Prüfkörper mit einer Heizrate von etwa 80 K/min weiter auf eine Temperatur von 1500°C aufgeheizt, bei dieser Temperatur etwa 5 Minuten lang gehalten und dann mit einer Abkühlrate von etwa 140 K/min auf eine Temperatur von etwa 1100°C abgekühlt wurden. Anschließend wurde die Heizkammer geöffnet.

Die Dichte der jeweils erhaltenen Oxidkeramikmaterialien sind in Tabelle 3 wiedergegeben.

Tabelle 3

| Material | Partielles Vakuum [mbar] | Dichte [g/cm$^3$] |
|---|---|---|
| IPS e.max ZirCAD LT A3 B45 | 50 | 6, 085 |
| | 100 | 6, 084 |
| | 200 | 6, 081 |

(fortgesetzt)

| Material | Partielles Vakuum [mbar] | Dichte [g/cm³] |
|---|---|---|
| Zenostar MT A3 | 50 | 6, 052 |
| | 100 | 6, 049 |
| | 200 | 6, 048 |

**Beispiel 4**

Gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT BL B45 oder IPS e.max ZirCAD LT A3 B45, Ivoclar Vivadent) bzw. aus kommerziell erhältlichen Oxidkeramik-Scheiben auf Basis von Zirkonoxid (Zenostar MT 3, Wieland Dental + Technik) erhaltene Prüfkörper wurden jeweils in einem Sinterofen mit SiC-Heizelement gesintert. Dazu wurden die Prüfkörper bei Raumtemperatur in die Heizkammer des Sinterofens eingebracht, die Heizkammer verschlossen und in der Heizkammer ein partielles Vakuum mit einem finalen Druck von etwa 50 bis 100 mbar erzeugt. Die Prüfkörper wurden mit einer Heizrate von etwa 180 K/min auf eine Temperatur von etwa 1050°C und weiter mit einer Heizrate von etwa 80 K/min auf eine Temperatur von etwa 1500°C aufgeheizt. Gemäß Tabelle 4 wurde die Heizkammer bei Erreichen einer Temperatur von 1250, 1300 bzw. 1350°C mit Frischluft bzw. Sauerstoff geflutet und anschließend während des restlichen Sinterprozesses kontinuierlich mit einer Strömungsgeschwindigkeit von etwa 5 l/min durchströmt. Bei einer Temperatur von etwa 1500°C wurden die Prüfkörper etwa 5 Minuten lang gehalten und dann mit einer Abkühlrate von etwa 140 K/min auf eine Temperatur von etwa 1100°C abgekühlt. Anschließend wurde die Heizkammer geöffnet.

Zum Vergleich wurde die obige Verfahrensweise wiederholt, wobei jedoch kein Vakuum erzeugt wurde, sondern die Heizkammer über die gesamte Dauer des Sinterprozesses kontinuierlich mit einer Strömungsgeschwindigkeit von etwa 5 l/min gemäß Tabelle 4 mit Frischluft oder Sauerstoff durchströmt wurde. In einem weiteren Vergleich wurde die obige Verfahrensweise wiederholt, wobei jedoch das partielle Vakuum mit einem finalen Druck von etwa 50 bis 100 mbar über die gesamte Dauer des Sinterprozesses aufrechterhalten wurde.

Die Dichte, mittlere Korngröße, Transluzenz und Farbkoordinaten der jeweils erhaltenen Oxidkeramikmaterialien sind in Tabelle 4 wiedergegeben.

Tabelle 4

| Material | Vakuum bis [°C] | Atmosphäre | Dichte [g/cm³] | Mittl. Korngröße [μm] * | CR [%] | L* | a* | b* |
|---|---|---|---|---|---|---|---|---|
| IPS e.max ZirCAD LT BL B45 | 1350 | Luft | 6,066 | 0,396 ± 0,075 | 88, 99 | 95,87 | -0,37 | 1,75 |
| | 1350 | O₂ | 6,057 | 0,375 ± 0,061 | 91,27 | 95,96 | -0,27 | 1,83 |
| | durchgehend Luft* | | 6,067 | 0,389 ± 0,077 | 92,54 | 96,14 | -0,31 | 1,81 |
| | durchgehend O₂* | | 6,047 | 0,386 ± 0,062 | 95,11 | 96,59 | -0,21 | 1,57 |
| IPS e.max ZirCAD LT A3 B45 | 1300 | Luft | 6,088 | | 97,57 | 83,58 | 4,34 | 17,13 |
| | durchgehend Luft* | | 6,076 | | 98,34 | 83,72 | 4,23 | 17,02 |
| | durchgehend O₂* | | 6,065 | | 100 | 85,49 | 2,83 | 15,15 |
| Zenostar MT A3 | 1250 | Luft | 6,052 | 0,584 ± 0,103 | 96,54 | 83,38 | 2,46 | 15,94 |
| | 1250 | O₂ | 6,052 | 0,543 ± 0,108 | 96,77 | 82,50 | 2,94 | 15,94 |
| | durchgehend Luft* | | 6,051 | 0,499 ± 0,131 | 98,31 | 84,93 | 1,57 | 14,04 |
| | durchgehend Vakuum* | | 6,051 | 0,528 ± 0,097 | 97,64 | 82,01 | 0,09 | 12,05 |
| * (Vergleich) ** Die nach DIN EN 623-3 gemessene zahlenmittlere Korngröße wurde nach M. I. Mendelson, J. Am. Ceram. Soc. 1969, 52(8), 443-446 mit 1,56 multipliziert, um den realen zahlenmittleren Durchmesser im dreidimensionalen Mikrogefüge zu erhalten. | | | | | | | | |

**Patentansprüche**

1. Verfahren zur Herstellung einer dentalen Restauration, bei dem ein Oxidkeramikmaterial

   (a) einer ersten Wärmebehandlung unterworfen wird,
   (b) einer zweiten Wärmebehandlung unterworfen wird und
   (c) abgekühlt wird,

   wobei die Wärmebehandlung in Schritt (a) bei niedrigerem Druck als die Wärmebehandlung in Schritt (b) erfolgt und die Wärmebehandlung in Schritt (b) in einer sauerstoffhaltigen Atmosphäre erfolgt
   und **dadurch gekennzeichnet, dass**
   die Wärmebehandlung in Schritt (a) bei einem Druck von weniger als 200 mbar erfolgt und/oder in Schritt (b) das Oxidkeramikmaterial in einem Schritt (b1) gegebenenfalls weiter aufgeheizt wird und in einem Schritt (b2) bei einer Temperatur im Bereich von 1100 bis 1600°C gehalten und gesintert wird.

2. Verfahren nach Anspruch 1, bei dem in Schritt (a) das Oxidkeramikmaterial auf eine Temperatur aufgeheizt wird, die im Bereich von 1100 bis 1600°C, insbesondere im Bereich von 1200 bis 1500°C, bevorzugt im Bereich von 1250 bis 1450°C und weiter bevorzugt im Bereich von 1300 bis 1400°C liegt und am meisten bevorzugt etwa 1350°C beträgt.

3. Verfahren nach Anspruch 1 oder 2, bei dem das Oxidkeramikmaterial in Schritt (a) mit einer Heizrate im Bereich von 10 bis 500 K/min, insbesondere 50 bis 250 K/min und bevorzugt 100 bis 200 K/min aufgeheizt wird.

4. Verfahren nach einem der Ansprüche 1 bis 3, bei dem die Wärmebehandlung in Schritt (a) bei einem Druck von weniger als 200 mbar, vorzugsweise weniger als 100 mbar und besonders bevorzugt weniger als 50 mbar und insbesondere bei einem Druck im Bereich von 0,1 bis 200 mbar, vorzugsweise im Bereich von 1 bis 150 mbar und besonders bevorzugt im Bereich von 50 bis 100 mbar erfolgt.

5. Verfahren nach einem der Ansprüche 1 bis 4, bei dem in Schritt (b) das Oxidkeramikmaterial (b1) gegebenenfalls weiter aufgeheizt wird und (b2) bei einer, vorzugsweise konstanten, Temperatur im Bereich von 1100 bis 1600°C, insbesondere im Bereich von 1300 bis 1600°C und bevorzugt im Bereich von 1350 bis 1550°C und besonders bevorzugt bei einer Temperatur von etwa 1500°C gehalten und gesintert wird.

6. Verfahren nach Anspruch 5, bei dem das Halten in Schritt (b2) für 1 bis 60 Minuten, insbesondere 2 bis 30 Minuten, bevorzugt 3 bis 15 Minuten und besonders bevorzugt etwa 5 Minuten erfolgt.

7. Verfahren nach einem der Ansprüche 1 bis 6, bei dem die Wärmebehandlung in Schritt (b) bei einem Druck von mehr als 500 mbar und insbesondere bei Umgebungsdruck erfolgt.

8. Verfahren nach einem der Ansprüche 1 bis 7, bei dem die Wärmebehandlung in Schritt (b) in Luft, mit Sauerstoff angereicherter Luft oder Sauerstoff erfolgt.

9. Verfahren nach Anspruch einem der Ansprüche 1 bis 8, bei dem während Schritt (b) die Heizkammer diskontinuierlich oder vorzugsweise kontinuierlich mit einer sauerstoffhaltigen Atmosphäre, vorzugsweise Luft, mit Sauerstoff angereicherter Luft oder Sauerstoff, durchströmt wird, insbesondere mit einer Strömungsgeschwindigkeit von 0,1 bis 50 l/min, bevorzugt 1 bis 20 l/min und besonders bevorzugt 4 bis 8 l/min.

10. Verfahren nach einem der Ansprüche 5 bis 9, bei dem das Oxidkeramikmaterial in Schritt (a) auf eine Temperatur aufgeheizt wird, die 0 bis 500 K, insbesondere 10 bis 250 K, bevorzugt 50 bis 200 K und besonders bevorzugt 100 bis 150 K unterhalb der Temperatur oder des Temperaturbereichs liegt, bei der/dem das Oxidkeramikmaterial in Schritt (b) gehalten wird.

11. Verfahren nach einem der Ansprüche 1 bis 10, bei dem das Oxidkeramikmaterial in Schritt (c) auf eine Temperatur abgekühlt wird, die im Bereich von 20 bis 1300°C, insbesondere im Bereich von 100 bis 1250°C und bevorzugt im Bereich von 1000 bis 1200°C liegt.

12. Verfahren nach einem der Ansprüche 1 bis 11, bei dem das Oxidkeramikmaterial auf Zirkonoxid und insbesondere auf polykristallinem tetragonalen Zirkonoxid (TZP) basiert.

**13.** Verfahren nach Anspruch 12, bei dem das Zirkonoxid mit $Y_2O_3$, $CeO_2$, MgO und/oder CaO stabilisiert ist und vorzugsweise mit 2 bis 12 Mol-%, insbesondere 3 bis 5 Mol-%, dieser Oxide, bezogen auf den Gehalt an Zirkonoxid, stabilisiert ist.

**14.** Verfahren nach einem der Ansprüche 1 bis 13, bei dem das Oxidkeramikmaterial eingefärbt ist und vorzugsweise mindestens zwei Schichten umfasst, die sich insbesondere in ihrer Farbe unterscheiden.

**15.** Verfahren nach einem der Ansprüche 1 bis 14, bei dem die dentale Restauration eine Brücke, ein Inlay, ein Onlay, eine Krone, ein Veneer, eine Schale oder ein Abutment ist und vorzugsweise zwei oder mehr Glieder umfasst.

**16.** Verwendung eines Oxidkeramikmaterials zur Herstellung einer dentalen Restauration, bei der das Oxidkeramikmaterial

> (a) einer ersten Wärmebehandlung unterworfen wird,
> (b) einer zweiten Wärmebehandlung unterworfen wird und
> (c) abgekühlt wird,
>
>> wobei die Wärmebehandlung in Schritt (a) bei niedrigerem Druck als die Wärmebehandlung in Schritt (b) erfolgt und die Wärmebehandlung in Schritt (b) in einer sauerstoffhaltigen Atmosphäre erfolgt und **dadurch gekennzeichnet, dass**
>> die Wärmebehandlung in Schritt (a) bei einem Druck von weniger als 200 mbar erfolgt und/oder in Schritt (b) das Oxidkeramikmaterial in einem Schritt (b1) gegebenenfalls weiter aufgeheizt wird und in einem Schritt (b2) bei einer Temperatur im Bereich von 1100 bis 1600°C gehalten und gesintert wird.

**Claims**

**1.** Process for the production of a dental restoration, in which an oxide ceramic material

> (a) is subjected to a first heat treatment,
> (b) is subjected to a second heat treatment and
> (c) is cooled,
>
>> wherein the heat treatment in step (a) is effected at lower pressure than the heat treatment in step (b) and the heat treatment in step (b) is effected in an oxygen-containing atmosphere
>> and **characterized in that**
>> the heat treatment in step (a) is effected at a pressure of less than 200 mbar and/or
>> in step (b) the oxide ceramic material in a step (b1) is optionally further heated and in a step (b2) is held and sintered at a temperature in the range of from 1100 to 1600°C.

**2.** Process according to claim 1, in which in step (a) the oxide ceramic material is heated to a temperature which lies in the range of from 1100 to 1600°C, in particular in the range of from 1200 to 1500°C, preferably in the range of from 1250 to 1450°C and further preferably in the range of from 1300 to 1400°C and is most preferably about 1350°C.

**3.** Process according to claim 1 or 2, in which in step (a) the oxide ceramic material is heated at a heating rate in the range of from 10 to 500 K/min, in particular 50 to 250 K/min and preferably 100 to 200 K/min.

**4.** Process according to any one of claims 1 to 3, in which the heat treatment in step (a) is effected at a pressure of less than 200 mbar, preferably less than 100 mbar and particularly preferred less than 50 mbar and in particular at a pressure in the range of from 0.1 to 200 mbar, preferably in the range of from 1 to 150 mbar and particularly preferred in the range of from 50 to 100 mbar.

**5.** Process according to any one of claims 1 to 4, in which in step (b) the oxide ceramic material is (b1) optionally further heated and (b2) held and sintered at a preferably constant temperature in the range of from 1100 to 1600°C, in particular in the range of from 1300 to 1600°C and preferably in the range of from 1350 to 1550°C and particularly preferred at a temperature of about 1500°C.

**6.** Process according to claim 5, in which the holding in step (b2) is effected for 1 to 60 minutes, in particular 2 to 30 minutes, preferably 3 to 15 minutes and particularly preferred about 5 minutes.

7. Process according to any one of claims 1 to 6, in which the heat treatment in step (b) is effected at a pressure of more than 500 mbar and in particular at ambient pressure.

8. Process according to any one of claims 1 to 7, in which the heat treatment in step (b) is effected in air, oxygen-enriched air or oxygen.

9. Process according to any one of claims 1 to 8, in which an oxygen-containing atmosphere, preferably air, oxygen-enriched air or oxygen, flows through the heating chamber during step (b) discontinuously or preferably continuously, in particular at a flow rate of from 0.1 to 50 l/min, preferably 1 to 20 l/min and particularly preferred 4 to 8 l/min.

10. Process according to any one of claims 5 to 9, in which in step (a) the oxide ceramic material is heated to a temperature which lies 0 to 500 K, in particular 10 to 250 K, preferably 50 to 200 K and particularly preferred 100 to 150 K below the temperature or the temperature range at or in which the oxide ceramic material is held in step (b) .

11. Process according to any one of claims 1 to 10, in which in step (c) the oxide ceramic material is cooled to a temperature which lies in the range of from 20 to 1300°C, in particular in the range of from 100 to 1250°C and preferably in the range of from 1000 to 1200°C.

12. Process according to any one of claims 1 to 11, in which the oxide ceramic material is based on zirconia and in particular on tetragonal zirconia polycrystal (TZP).

13. Process according to claim 12, in which the zirconia is stabilized with $Y_2O_3$, $CeO_2$, MgO and/or CaO and is preferably stabilized with 2 to 12 mol-%, in particular 3 to 5 mol-%, of these oxides, relative to the zirconia content.

14. Process according to any one of claims 1 to 13, in which the oxide ceramic material is coloured and preferably comprises at least two layers which differ in particular in their colour.

15. Process according to any one of claims 1 to 14, in which the dental restoration is a bridge, an inlay, an onlay, a crown, a veneer, a facet or an abutment and preferably comprises two or more units.

16. Use of an oxide ceramic material for the production of a dental restoration, in which the oxide ceramic material

    (a) is subjected to a first heat treatment,
    (b) is subjected to a second heat treatment and
    (c) is cooled,

    wherein the heat treatment in step (a) is effected at lower pressure than the heat treatment in step (b) and the heat treatment in step (b) is effected in an oxygen-containing atmosphere
    and **characterized in that**
    the heat treatment in step (a) is effected at a pressure of less than 200 mbar and/or
    in step (b) the oxide ceramic material in a step (b1) is optionally further heated and in a step (b2) is held and sintered at a temperature in the range of from 1100 to 1600°C.

**Revendications**

1. Procédé de fabrication d'une pièce de restauration dentaire, dans lequel un matériau céramique d'oxyde

    a) est soumis à un premier traitement thermique,
    b) est soumis à un deuxième traitement thermique,
    c) et est refroidi,

    étant entendu que le traitement thermique de l'étape (a) est effectué sous une plus basse pression que le traitement thermique de l'étape (b) et que le traitement thermique de l'étape (b) est effectué dans une atmosphère contenant de l'oxygène,
    et qui est **caractérisé en ce que** le traitement thermique de l'étape (a) est effectué sous une pression valant moins de 200 mbar,
et/ou **en ce que** dans l'étape (b), le matériau céramique d'oxyde, dans une étape (b1), est éventuellement chauffé encore davantage, et dans une étape (b2), est maintenu à une température située dans l'intervalle allant de 1100 à 1600°C et fritté.

2. Procédé conforme à la revendication 1, dans lequel, dans l'étape (a), le matériau céramique d'oxyde est chauffé à une température qui se situe dans l'intervalle allant de 1100 à 1600 °C, en particulier dans l'intervalle allant de 1200 à 1500 °C, de préférence dans l'intervalle allant de 1250 à 1450 °C, mieux encore dans l'intervalle allant de 1300 à 1400 °C, et surtout aux environs de 1350 °C.

3. Procédé conforme à la revendication 1 ou 2, dans lequel le matériau céramique d'oxyde, dans l'étape (a), est chauffé à une vitesse de chauffage située dans l'intervalle allant de 10 à 500 K/min, en particulier de 50 à 250 K/min, et de préférence de 100 à 200 K/min.

4. Procédé conforme à l'une des revendications 1 à 3, dans lequel le traitement thermique de l'étape (a) est effectué sous une pression valant moins de 200 mbar, de préférence moins de 100 mbar et mieux encore moins de 50 mbar, et en particulier sous une pression située dans l'intervalle allant de 0,1 à 200 mbar, de préférence dans l'intervalle allant de 1 à 150 mbar, et mieux encore dans l'intervalle allant de 50 à 100 mbar.

5. Procédé conforme à l'une des revendications 1 à 4, dans lequel, dans l'étape (b), le matériau céramique d'oxyde, (b1) est éventuellement chauffé encore davantage et (b2) est maintenu à une température, de préférence constante, située dans l'intervalle allant de 1100 à 1600°C, en particulier dans l'intervalle allant de 1300 à 1600 °C, de préférence dans l'intervalle allant de 1350 à 1550 °C, et mieux encore à une température située aux environs de 1500 °C, et fritté.

6. Procédé conforme à la revendication 5, dans lequel, dans l'étape (b2), le maintien en température dure de 1 à 60 minutes, en particulier de 2 à 30 minutes, de préférence de 3 à 15 minutes, et mieux encore, environ 5 minutes.

7. Procédé conforme à l'une des revendications 1 à 6, dans lequel le traitement thermique de l'étape (b) est effectué sous une pression valant plus de 500 mbar, et en particulier sous la pression ambiante.

8. Procédé conforme à l'une des revendications 1 à 7, dans lequel le traitement thermique de l'étape (b) est effectué dans l'air, dans de l'air enrichi en oxygène, ou dans de l'oxygène.

9. Procédé conforme à l'une des revendications 1 à 8, dans lequel, pendant l'étape (b), la chambre de chauffage est traversée, en discontinu ou de préférence en continu, par un courant de gaz contenant de l'oxygène, de préférence un courant d'air, d'air enrichi en oxygène ou d'oxygène, à un débit valant en particulier de 0,1 à 50 L/min, de préférence de 1 à 20 L/min et mieux encore de 4 à 8 L/min.

10. Procédé conforme à l'une des revendications 5 à 9, dans lequel, dans l'étape (a), le matériau céramique d'oxyde est chauffé à une température qui se situe de 0 à 500 K, en particulier de 10 à 250 K, de préférence de 50 à 200 K, et mieux encore de 100 à 150 K au-dessous de la température ou de l'intervalle de température à laquelle ou dans lequel le matériau céramique d'oxyde est maintenu dans l'étape (b).

11. Procédé conforme à l'une des revendications 1 à 10, dans lequel, dans l'étape (c), le matériau céramique d'oxyde est refroidi à une température qui se situe dans l'intervalle allant de 20 à 1300 °C, en particulier dans l'intervalle allant de 100 à 1250 °C, et de préférence dans l'intervalle allant de 1000 à 1200 °C.

12. Procédé conforme à l'une des revendications 1 à 11, dans lequel le matériau céramique d'oxyde est à base d'oxyde de zirconium et en particulier, à base d'oxyde de zirconium tétragonal polycristallin (TZP).

13. Procédé conforme à la revendication 12, dans lequel l'oxyde de zirconium est stabilisé avec $Y_2O_3$, $CeO_2$, MgO et/ou CaO, de préférence avec de 2 à 12 % en moles et en particulier de 3 à 5 % en moles de ces oxydes, par rapport à la teneur en oxyde de zirconium.

14. Procédé conforme à l'une des revendications 1 à 13, dans lequel le matériau céramique d'oxyde est coloré dans la masse et comprend de préférence au moins deux couches qui diffèrent en particulier par leur couleur.

15. Procédé conforme à l'une des revendications 1 à 14, dans lequel la pièce de restauration dentaire est un bridge, un

# EP 3 659 548 B1

incrustation in-lay ou on-lay, une couronne, une facette, une coquille ou un pilier, et comprend de préférence deux maillons ou plus.

16. Utilisation d'un matériau céramique d'oxyde pour la fabrication d'une pièce de restauration dentaire, dans laquelle le matériau céramique d'oxyde

    a) est soumis à un premier traitement thermique,
    b) est soumis à un deuxième traitement thermique,
    c) et est refroidi,

    étant entendu que le traitement thermique de l'étape (a) est effectué sous une plus basse pression que le traitement thermique de l'étape (b) et que le traitement thermique de l'étape (b) est effectué dans une atmosphère contenant de l'oxygène,
    et qui est **caractérisée en ce que** le traitement thermique de l'étape (a) est effectué sous une pression valant moins de 200 mbar,
    et/ou **en ce que** dans l'étape (b), le matériau céramique d'oxyde, dans une étape (b1), est éventuellement chauffé encore davantage, et dans une étape (b2), est maintenu à une température située dans l'intervalle allant de 1100 à 1600°C et fritté.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2098188 A1
- EP 2101133 A1
- WO 2012057829 A2
- WO 2015091744 A1
- WO 2015121364 A1
- WO 2011020688 A1

- EP 2703760 A1
- EP 2022442 A1
- WO 2015200017 A1
- EP 0853938 A1
- US 2017349494 A1

**In der Beschreibung aufgeführte Nicht-Patentliteratur**

- **M. I. MENDELSON**. *J. Am. Ceram. Soc.*, 1969, vol. 52 (8), 443-446